# NOUVEAU FASCICULE DE BREVET EUROPEEN

(11) **EP 1 896 245 B2**
(45) Date de publication et mention de la décision concernant l'opposition: **26.06.2013**
(45) Mention de la délivrance du brevet: 13.05.2009
(21) Numéro de dépôt: 06763581.3
(22) Date de dépôt: 08.06.2006
(51) Int. Cl.: B29C 49/42, B29C 49/46

(54) **PROCEDE DE STERILISATION DE PREFORMES ET INSTALLATION PRODUISANT DES BOUTEILLES STERILES A PARTIR DE CES PREFORMES**
VERFAHREN ZUR STERILISATION VON VORFORMLINGEN UND ANLAGE ZUR HERSTELLUNG VON STERILEN KÖRPERS AUS DIESEN VORFORMLINGEN
METHOD FOR STERILIZING PREFORMS AND INSTALLATION FOR PRODUCING STERILE BODIES FROM THESE PREFORMS

(30) Priorité: 24.06.2005 FR 0551752
(43) Date de publication de la demande: 12.03.2008
(73) Titulaire: Sidel Participations, 76930 Octeville Sur Mer (FR)
(72) Inventeur: ADRIANSENS, Eric c/o Sidel Participations, F-76930 Octeville Sur Mer (FR); HEBERT, Stéphane c/o Sidel Participations, F-76930 Octeville Sur Mer (FR)
(74) Mandataire: Kohn, Philippe
(86) Numéro de dépôt international: PCT/EP2006/063006
(87) Numéro de publication internationale: WO 2006/136499

(56) Documents cités:
- EP-A- 1 122 168
- WO-A-99/03667
- DE-A1- 19 949 692
- FR-A1- 2 766 169
- US-A- 4 512 951
- US-B1- 6 692 684
- PATENT ABSTRACTS OF JAPAN vol. 016, no. 127 (M-1227), 31 mars 1992 (1992-03-31) & JP 03 290226 A (DAINIPPON PRINTING CO LTD), 19 décembre 1991 (1991-12-19)

## Description

L'invention concerne un procédé de stérilisation de préformes et une installation produisant des bouteilles stériles à partir des préformes stérilisées selon ce procédé.

L'invention concerne plus particulièrement un procédé de stérilisation d'une préforme en matière plastique destinée à être moulée, notamment par soufflage, dans une installation produisant des bouteilles stériles.

L'invention concerne aussi une installation pour produire des bouteilles stériles à partir de préformes en matière plastique mettant en oeuvre le procédé de stérilisation de préformes selon l'invention, du type dans lequel les préformes sont convoyées à l'intérieur de l'installation selon un flot continu qui circule de l'amont vers l'aval, et du type comportant successivement au moins :
- une unité de stérilisation comportant au moins un poste de projection pourvu d'au moins une buse pour projeter, en cours de traitement, un flux de produit stérilisant sous forme d'un jet de vapeur vers le col de chaque préforme à stériliser ;
- une unité de mise en forme comportant un poste de conditionnement thermique qui comporte un four destiné à porter chaque préforme à une température de moulage, et un poste de moulage apte à produire une bouteille stérile.

Un tel type d'installation est décrit et représenté notamment dans le document US-A-2001/0010145.

Ce type d'installation présente l'inconvénient de nécessiter un débit de produit stérilisant et/ou une pression d'injection du produit stérilisant de valeurs importantes pour réussir à couvrir complètement les parois internes des préformes de manière à stériliser complètement l'intérieur des préformes.

Par conséquent, la consommation de l'installation en produit stérilisant est donc importante et l'opération de stérilisation coûteuse.

De plus, l'utilisation d'un débit important de produit stérilisant peut conduire au dépôt de gouttelettes résiduelles de produit stérilisant sur les parois internes des préformes. Or, lors du chauffage des préformes, ces gouttelettes produisent un effet loupe sur les rayonnements thermiques de chauffage, ce qui conduit à l'apparition de taches sur les parois des bouteilles issues des préformes concernées.

En effet, dans les installations de l'état de la technique, le produit stérilisant est généralement pulvérisé sous la forme d'un brouillard et avec une pression obtenue en comprimant un gaz, tel que de l'air comprimé à des pressions d'environ 2 à 3 bars, qui est stérilisé et chauffé, par exemple à une température de l'ordre de 130°C de manière à activer thermiquement le produit stérilisant.

C'est la raison pour laquelle, les gouttelettes de produit stérilisant forment un excédent qui n'est pas entièrement vaporisé lors du chauffage de telle sorte que chaque gouttelette provoque localement une attaque chimique du matériau de la préforme, généralement en polyéthylène téréphtalate (PET), par le produit stérilisant.

Ce phénomène conduit à l'apparition de taches sur les parois des bouteilles, ce défaut d'aspect étant encore parfois appelé "peau d'orange".

De plus, les parois de la préformes ne sont pas uniformément recouvertes par les gouttelettes de produit stérilisant de sorte que des zones non stérilisées subsistent entre chacune des gouttelettes, sur la surface des parois interne et/ou externe de la préforme.

De surcroît, suivant le profil de la paroi interne de chaque préforme, il n'est pas toujours possible d'atteindre à coup sûr le fond des préformes, même avec un débit et/ou une pression de valeurs importantes, en raison de la création d'un bouchon de surpression au fond des préformes.

Un tel type d'installation est encore décrit et représenté dans le document WO-A2-99/03667 qui concerne un procédé pour la fabrication de récipients stériles en matière plastique et une installation pour sa mise en oeuvre.

L'installation comporte un dispositif d'amenée des préformes à des moyens de chauffage en amont duquel les préformes sont traitées, au moins à l'intérieur des préformes, par des moyens de stérilisation.

Les moyens de stérilisation comprennent notamment un produit de stérilisation, tel qu'une solution chimique de peroxyde d'hydrogène (H₂O₂) activable thermiquement, notamment par la chaleur des moyens de chauffage, avant d'être éliminé par évaporation.

Les moyens de stérilisation comportent pour ce faire un pulvérisateur constitué par un pistolet de vaporisation qui permet de mouiller l'intérieur des préformes avec du produit de stérilisation "froid", c'est-à-dire n'ayant pas été préalablement chauffé et se trouvant donc à l'état liquide.

Dans l'installation décrite dans ce document, le pistolet est typiquement un pistolet de type bi-fluide comportant une buse de liquide et une buse d'air formant un ensemble de projection circulaire qui est susceptible d'être placé au-dessus du trajet des préformes de manière à pulvériser un brouillard de produit de stérilisation.

Le brouillard de produit de stérilisation est formé d'un nuage de gouttelettes qui est projeté par le pistolet vers l'intérieur de la préforme suivant un écoulement de type turbulent.

On a constaté qu'un tel écoulement turbulent de produit de stérilisation ou stérilisant à l'intérieur de la préforme conduit à la formation d'un ensemble de gouttelettes qui ne sont reparties de manière homogène sur la paroi interne de la préforme.

En effet, l'utilisation d'un pistolet de pulvérisation est caractérisé notamment par un débit important de produit stérilisant qui est obtenu en comprimant un gaz, tel que de l'air comprimé à des pressions d'environ 2 à 3 bars, de sorte qu'il produit un écoulement turbulent.

Or, l'écoulement turbulent conduit au dépôt non homogène de gouttelettes résiduelles de produit stérilisant sur les parois internes des préformes. De plus, les gouttelettes de produit stérilisant forment un excédent qui n'est pas entièrement vaporisé lors du chauffage.

Ces gouttelettes de produit stérilisant provoquent donc d'une part localement une attaque chimique du matériau de la préforme, généralement en polyéthylène téréphtalate (PET), et, d'autre part, lors du chauffage des préformes, produisent un effet loupe sur les rayonnements thermiques de chauffage, ce qui provoque l'apparition de taches sur les parois des bouteilles issues des préformes traitées.

L'apparition de telles taches sur les parois des bouteilles, est un défaut d'aspect du produit, qui est encore parfois appelé "aspect peau d'orange".

La présente invention vise donc à remédier à ces inconvénients et à proposer notamment un procédé de stérilisation et une installation qui soient simples et économiques.

Dans ce but, l'invention propose un procédé de stérilisation du type décrit précédemment, **caractérisé en ce qu**'il comporte au moins les étapes consistant successivement :
(a) à vérifier que la préforme est à une température inférieure à la température de condensation du produit stérilisant ;
(b) à projeter un flux de produit stérilisant sous forme d'un jet de vapeur vers la préforme de manière à provoquer le dépôt par condensation d'un film de buée de produit stérilisant sensiblement uniforme sur au moins une paroi interne de la préforme à stériliser ; et
(c) à chauffer par rayonnement la préforme ainsi traitée pour la porter à une température supérieure ou égale à la température d'activation du produit stérilisant de manière à stériliser au moins la paroi interne de la préforme.

Avantageusement, le procédé de stérilisation comporte une étape supplémentaire de stérilisation (d) consistant à projeter un flux de produit stérilisant sous forme d'un jet de vapeur vers la préforme préalablement chauffée à une température déterminée.

Avantageusement, la température de chauffage de la préforme est sensiblement égale à la température de moulage par soufflage de la préforme, ladite température de moulage étant respectivement supérieure à la température d'activation et à la température d'évaporation pour éliminer le produit stérilisant par évaporation.

L'invention propose aussi une installation du type décrit précédemment, **caractérisée en ce que,** au premier poste de projection, le flux de produit stérilisant est vaporisé sur chaque préforme dont la température est inférieure à la température de condensation du produit stérilisant de manière qu'un film de buée de produit stérilisant, sensiblement uniforme, se dépose par condensation sur la paroi interne à stériliser, et en ce que l'unité de stérilisation comporte un poste d'activation du produit stérilisant comportant des moyens de chauffage par rayonnement constituant le four pour chauffer chaque préforme ainsi traitée jusqu'à une température supérieure ou égale à la température d'activation de manière à stériliser au moins la paroi interne de la préforme et en ce que l'axe moyen de projection (A2) de la buse de vaporisation du produit stérilisant vers le col est globalement parallèle à l'axe (A1) de la préforme en cours de traitement et excentré radialement, par rapport à l'axe (A1) de la préforme, de manière à produire un écoulement de produit stérilisant de type laminaire à l'intérieur de la préforme.

Selon d'autres caractéristiques de l'invention :
- les moyens de chauffage du poste d'activation sont constitués par le four du poste de conditionnement thermique de manière que chaque préforme est chauffée à une température de moulage qui est respectivement supérieure à la température d'activation du produit stérilisant et à la température d'évaporation pour éliminer le produit stérilisant par évaporation ;
- le poste de moulage de l'unité de mise en forme comporte au moins un moule et au moins un dispositif de soufflage associé qui soumet chaque préforme à une surpression interne de manière que chaque préforme stérilisée prenne la forme de l'empreinte du moule pour produire la bouteille stérile ;
- dans l'unité de stérilisation, les préformes sont globalement alignées suivant une direction longitudinale de défilement (X1), et sont disposées en position debout, parallèlement les unes à côté des autres, et en ce que l'axe de projection (A2) de la buse est décalé radialement suivant une direction sensiblement orthogonale, par rapport à la direction de défilement (X1) ;
- le produit stérilisant est constitué d'un composé contenant du peroxyde d'hydrogène ou de peroxyde d'hydrogène vaporisé qui est activé en chauffant la préforme au-delà de la température d'activation ;
- le produit stérilisant comportant du peroxyde d'hydrogène est projeté à l'état de vapeur à une température supérieure à cent six degrés Celsius ;
- l'installation est du type comportant au moins un dispositif de transport pourvu de moyens de préhension du col de chaque préforme pour convoyer le flot de préformes dans le four, qui comporte au moins un moyen, tel qu'un noyau interne, qui pénètre axialement à l'intérieur du col de chaque préforme en obstruant tout ou partie de l'ouverture intérieure délimitée par le col de manière à accroître le degré de stérilisation par augmentation de la durée d'exposition de la paroi interne de la préforme avec le produit stérilisant ;
- l'unité de stérilisation comporte, en aval du four, un autre poste de projection, dit deuxième poste, qui est destiné à projeter un second flux de produit stérilisant vers chaque préforme préalablement chauffée à une température déterminée qui est au moins supérieure à la température d'activation et à la température d'évaporation du produit stérilisant.

D'autres caractéristiques et avantages de l'invention apparaîtront à la lecture de la description détaillée qui suit pour la compréhension de laquelle on se reportera aux dessins annexés dans lesquels :
- la figure 1 représente schématiquement un premier exemple de réalisation d'une installation produisant des bouteilles stériles par soufflage et comportant une unité de stérilisation mettant en oeuvre le procédé de stérilisation selon l'invention ;
- la figure 2 est une vue en coupe axiale suivant le plan de coupe 2-2 qui représente schématiquement une préforme dans l'unité de stérilisation de l'installation de la figure 1 ;
- la figure 3 est une vue de dessus qui représente une série de préformes au poste de projection de l'unité de stérilisation ;
- la figure 4 représente schématiquement un deuxième exemple de réalisation d'une installation produisant des bouteilles stériles par soufflage et comportant une unité de stérilisation qui comporte un premier poste de projection mettant en oeuvre le procédé de stérilisation selon l'invention et un deuxième poste de projection.

Dans la suite de la description, des éléments similaires ou identiques seront désignés par les mêmes références.

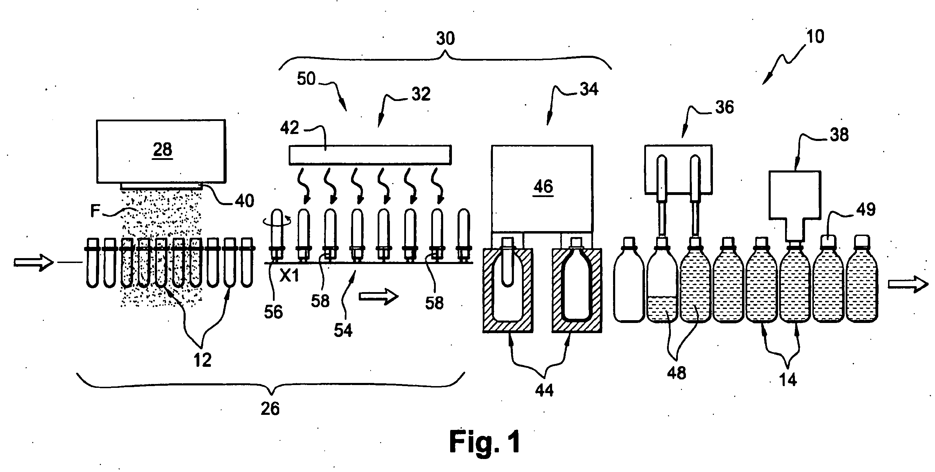

Sur la figure 1, on a représenté une installation 10 produisant des bouteilles 14, en particulier des bouteilles stériles ou aseptisées, qui sont avantageusement obtenues par soufflage à partir de préformes 12 en matière plastique.

Ce type d'installation 10 est utilisé, par exemple, pour la fabrication de bouteilles 14 en matière plastique telle que du polyéthylène téréphtalate (PET).

Chaque préforme 12 a globalement la forme d'un tube qui est fermé à une extrémité et dont l'autre extrémité possède déjà la forme définitive du col 16 de la bouteille 14.

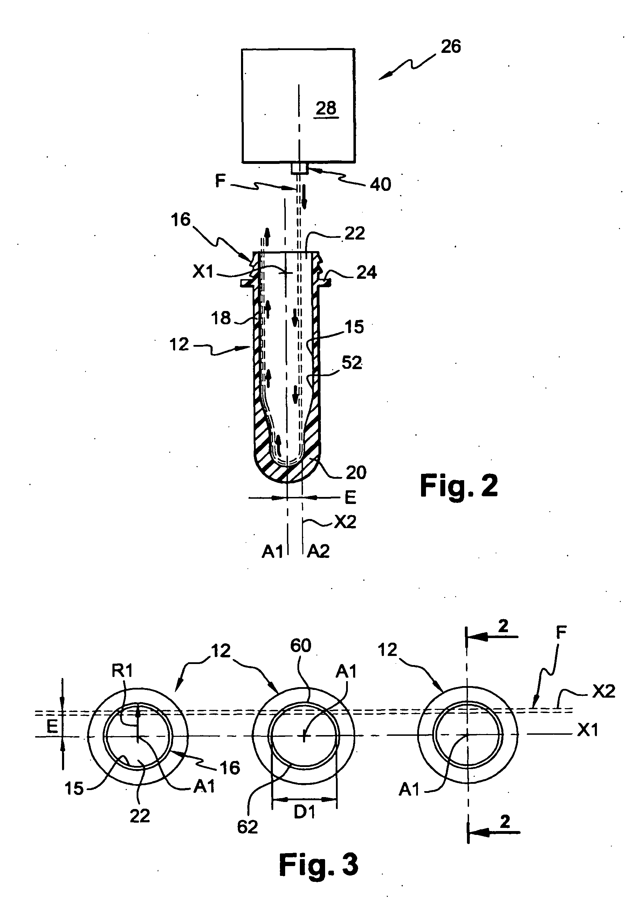

Sur la figure 2, une préforme 12 est représentée, à titre non limitatif, avec l'axe A1 de son corps 18 cylindrique qui s'étend verticalement et qui est confondu avec l'axe du col 16.

L'extrémité inférieure 20 de la préforme 12 est fermée, tandis que son extrémité supérieure forme le col 16 qui délimite une ouverture intérieure 22 et qui est ici pourvu d'une collerette 24 radiale externe.

Les préformes 12 sont généralement réalisées selon un procédé de moulage par injection et sont par exemple moulées sur un autre lieu que celui où se trouve l'installation 10.

Pour certaines applications, les bouteilles 14 obtenues à partir des préformes 12 doivent présenter un certain degré de stérilité, c'est la raison pour laquelle on procède généralement à une opération de stérilisation des préformes 12 dans l'installation 10 de production des bouteilles 14.

Plus précisément, l'opération de stérilisation concerne généralement le col 16 et la paroi 15 interne de la préforme 12 correspondant à la paroi interne délimitant le volume intérieur de la bouteille 14 destiné à être rempli.

La figure 1 représente un premier exemple de réalisation d'une installation 10 mettant en oeuvre le procédé de stérilisation de préformes 12 selon l'invention.

Conventionnellement, les préformes 12 sont convoyées à l'intérieur d'une telle installation 10 selon un flot continu qui circule de l'amont vers l'aval, c'est-à-dire de la gauche vers la droite en considérant la figure 1.

L'installation 10 comporte, de l'amont vers l'aval, une unité de stérilisation 26 des préformes 12 comportant au moins un poste de projection 28 et une unité de mise en forme 30 des préformes 12 stérilisées comportant un poste de conditionnement thermique 32 et un poste de moulage 34.

Avantageusement, l'installation 10 comporte aussi, à la suite du poste de moulage 34 de l'unité de mise en forme 30, une unité de remplissage 36 et une unité de bouchage 38.

De préférence, l'unité de stérilisation 26 comporte des moyens (non représentés) de préparation du produit stérilisant vaporisé pour alimenter le poste de projection 28.

Le poste de projection 28 de l'unité de stérilisation 26 est pourvu d'au moins une buse 40 qui projette, en cours de traitement, un flux F de produit stérilisant sous la forme d'un jet de vapeur, ici vers le col 16 de chaque préforme 12 à stériliser.

Ainsi, les moyens de préparation du produit stérilisant comportent par exemple des moyens de chauffage (non représentés) du produit stérilisant et une source d'air (non représentée), avantageusement comprimé et/ou stérilisé par tout moyen approprié, qui est prévue pour propulser le produit stérilisant vaporisé à travers la buse 40.

Avantageusement, l'air comprimé est déshydraté et circule à faible vitesse selon un écoulement directif de manière à constituer un vecteur pour la vapeur de produit stérilisant.

De préférence, le produit stérilisant est constitué d'un composé contenant du peroxyde d'hydrogène ou de peroxyde d'hydrogène vaporisé (H₂O₂) qui, au poste de projection 28, est projeté vers les préformes 12 sous la forme d'un jet de gaz comportant du produit stérilisant à l'état de vapeur, avantageusement un jet de vapeur sèche.

Conformément au procédé de stérilisation selon l'invention, pour stériliser notamment la paroi 15 interne de la préforme 12, on procède successivement au moins aux étapes suivantes :
(a) vérifier que la préforme 12 est à une température T1 inférieure à la température de condensation Tc du produit stérilisant ;
(b) projeter un flux F de produit stérilisant sous forme d'un jet de vapeur vers le col 16 de la préforme 12 de manière à provoquer le dépôt par condensation d'un film de buée de produit stérilisant sensiblement uniforme au moins sur la paroi 15 interne de la préforme 12 à stériliser ; et
(c) chauffer par rayonnement la préforme 12 ainsi traitée afin de porter la préforme 12 à une température T2 supérieure ou égale à la température d'activation Ta du produit stérilisant de manière à stériliser au moins la paroi 15 interne de la préforme 12.

Avantageusement, la température T2 est aussi supérieure à la température d'évaporation Te du produit stérilisant de manière à provoquer son élimination par évaporation simultanément à son activation thermique par rayonnement.

Avantageusement, la température de condensation Tc du peroxyde d'hydrogène est telle que, lorsque la préforme 12 est à une température sensiblement égale à la température ambiante de l'installation 10, par exemple comprise entre 7°C et 35°C, on obtient facilement une bonne condensation du produit stérilisant vaporisé.

De plus, la vérification de température selon l'étape (a) s'en trouve alors simplifiée. En effet, il n'est pas nécessaire de modifier la température de la préforme 12 par chauffage ou refroidissement, pour obtenir une condensation du produit stérilisant dans la paroi 15 interne de la préforme.

L'unité de stérilisation 26 sera décrite de manière plus détaillée par la suite, en relation avec les caractéristiques du procédé selon l'invention.

Le poste de conditionnement thermique 32 de l'unité de mise en forme 30 comporte au moins un four 42 qui est conventionnellement destiné à chauffer les préformes 12 pour les porter à une température appropriée au moulage.

Le poste de moulage 34 comporte au moins un moule 44 et ici au moins un dispositif de soufflage 46 qui soumet chaque préforme 12 à une surpression interne de manière qu'elle prenne la forme de l'empreinte du moule 44 ce qui produit une bouteille 14 stérile.

Le poste de moulage 34 peut aussi comporter des moyens d'élongation (non représentés) qui étirent la préforme 12 vers le fond du moule 44, pendant l'opération de moulage.

L'unité de remplissage 36 injecte le produit 48 à conditionner dans les bouteilles 14 issues du poste de moulage 34, puis l'unité de bouchage 38 ferme hermétiquement les bouteilles 14 stériles remplies avec une capsule 49 appropriée, en variante avec un opercule et/ou un bouchon.

On décrira maintenant, de manière plus détaillée, l'unité de stérilisation 26 de l'installation 10 selon les enseignements de l'invention.

Selon l'invention, l'unité de stérilisation 26 de l'installation 10 comporte d'une part le poste de projection 28 de produit stérilisant pour réaliser l'étape (b) et, d'autre part, un poste d'activation 50 du produit stérilisant pour réaliser l'étape (c).

Ainsi, au poste de projection 28, le flux (F) de produit stérilisant est vaporisé sur chaque préforme 12 dont la température T1 est inférieure à la température de condensation Tc du produit stérilisant de manière qu'un film de buée 52 de produit stérilisant se dépose uniformément par condensation au moins sur la paroi 15 interne et le col 16 de la préforme 12 à stériliser.

La concentration de la vapeur en produit stérilisant est, par exemple, sensiblement égale à vingt-cinq pourcents.

Le poste d'activation 50 du produit stérilisant comporte des moyens de chauffage par rayonnement, tel qu'un rayonnement de type infrarouge, pour porter par chauffage chaque préforme 12 préalablement traitée à l'étape (a) jusqu'à une température T2 supérieure ou égale à la température d'activation Ta de manière à stériliser notamment la paroi 15 interne de la préforme 12.

A la sortie de la buse 40, la vapeur contenant le produit stérilisant atteint une température T donnée sensiblement supérieure à la température d'évaporation Te du produit stérilisant de sorte que la vapeur de produit stérilisant se condense instantanément sur la préforme 12.

Dans le cas d'utilisation de peroxyde d'hydrogène (H₂O₂), la température T en sortie de buse est avantageusement supérieure à cent six degrés Celsius (106°C), de préférence comprise entre cent dix degrés Celsius (110°C) et cent vingt degrés Celsius (120°C).

Lorsque cette vapeur entre en contact avec chaque préforme 12, qui est plus froide, le produit stérilisant vaporisé se condense de manière que l'ensemble de la préforme 12, en particulier sur la paroi 15 interne, s'embuent alors d'un film de buée 52 de produit stérilisant.

La buse 40 est ici réalisée de manière que le film de buée 52 sensiblement uniforme se dépose principalement sur le col 16 et sur l'ensemble de la surface de la paroi 15 interne de la préforme 12.

Avantageusement, le dépôt par condensation suivant le film de buée 52 uniforme de produit stérile permet, par rapport à l'état de la technique, d'éliminer tout risque d'apparition de taches et d'aspect "peau d'orange".

Les moyens de chauffage du poste d'activation du produit stérilisant sont par exemple constitués par un four de chauffage par rayonnement comportant des éléments chauffants.

Avantageusement, les moyens de chauffage du poste d'activation 50 sont ici constitués par le four 42 du poste de conditionnement thermique 32.

Ainsi, la préforme 12 est chauffée jusqu'à atteindre une température de moulage Tm qui selon le type de préformes pourra varier entre 95°C et 135°C qui est ici respectivement supérieure à la température d'activation Ta du produit stérilisant et à la température d'évaporation Te permettant conjointement l'élimination par évaporation du produit stérilisant.

En effet, la température d'activation Ta du peroxyde d'hydrogène (H₂O₂) est d'environ soixante dix degrés Celsius (70°C), c'est-à-dire ici une température inférieure à la température de moulage Tm puisque la température de moulage Tm est par exemple comprise entre quatre-vingt-quinze degrés Celsius (95°C) et cent trente cinq degrés Celsius (135°C).

Bien entendu, la température de moulage Tm dépend du type de préforme 12 lequel est déterminé en fonction des applications et des récipients ou bouteilles 14.

De manière connue, le four 42 a pour fonction dans l'unité de conditionnement thermique 32 de réchauffer les préformes 12 à une température supérieure à la température de moulage (95°C à 135°C) afin de pouvoir réaliser ensuite l'opération de moulage au poste de moulage 34, notamment par soufflage de la préforme 12 stérilisée.

Cependant, le four 42 de chauffage par rayonnement étant ici commun aux postes d'activation 50 et de conditionnement thermique 32, il exerce alors une double fonction dans l'installation 10.

En effet, le four 42 provoque par chauffage d'une part l'activation du produit stérilisant condensé ce qui a un effet bactéricide immédiat sur la paroi 15 interne des préformes 12 et, d'autre part, le chauffage de la préforme 12 pour procéder à son moulage par soufflage pour afin d'obtenir la bouteille 14 stérile désirée.

De surcroît, comme la température de moulage Tm est supérieure à la température d'évaporation Te, l'élimination du produit stérilisant se fait automatiquement par évaporation sans requérir de moyens supplémentaires.

De manière connue et non représentée, un tel four 42 de chauffage comporte généralement un tunnel longitudinal de chauffage le long duquel les préformes 12 sont transportées par un dispositif de transport 54, aussi appelé tournette, entre une première extrémité du tunnel où les préformes 12 pénètrent froides avant de ressortir par la seconde extrémité du tunnel chauffées ou réchauffées, prêtes pour l'opération de moulage/soufflage.

Par ailleurs, une paroi du tunnel est équipée de moyens de chauffage à rayonnement, tels que des lampes infrarouge, tandis que l'autre paroi est munie d'orifices d'aération pour permettre le passage d'air soufflé afin de favoriser un chauffage homogène dans toute l'épaisseur de la préforme 12 sans surchauffer la couche de matière superficielle.

En effet, l'air soufflé permet d'évacuer la chaleur de convection provoquée par les moyens de chauffage pour favoriser la pénétration du rayonnement qu'ils produisent dans l'épaisseur de la matière constituant la préforme 12.

Pour plus de détails sur de tels fours 42 de chauffage de préformes, on se reportera par exemple aux documents suivants : EP-A-0.620.099 ou EP-A-0.564.354.

De préférence, le four 42 comporte des moyens de protection notamment pour limiter la corrosion des parties ou pièces exposées au produit stérile qui s'évaporant des préformes 12 est ainsi soufflée à l'intérieur du tunnel.

Avantageusement, le dispositif de transport 54 est pourvu de moyens de préhension 56 du col 16 de chaque préforme 12 pour convoyer le flot de préformes 12 dans le four 42.

Le document WO-A-00/48819, auquel on se reportera pour plus de détails, décrit un exemple de dispositif de transport 54 de ce type comportant avantageusement des moyens pour mettre les préformes 12 en rotation sur elles-mêmes pendant leur circulation dans le four 42 de manière à assurer le chauffage en profondeur de la préforme 12, c'est-à-dire tant de l'extrémité inférieure 20 formant fond que du corps 18 cylindrique.

Le dispositif de transport 54 comporte au moins un moyen, tel qu'un noyau interne 58, aussi appelé nez de tournette, qui pénètre axialement à l'intérieur du col 16 de chaque préforme 12 en obstruant tout ou partie de l'ouverture intérieure 22 délimitée par le col 16 de manière à accroître le degré de stérilisation par augmentation de la durée d'exposition de la paroi 15 interne de la préforme 12 avec le produit stérilisant.

Cependant dans le cas d'une installation 10 telle que représentée à la figure 1, l'air soufflé sur chaque préforme 12 dans le four 42 provoque l'élimination de tout ou partie du film de buée 52 susceptible de s'être aussi déposé par condensation sur la paroi externe de la préforme 12 au poste de projection 28 de l'unité de stérilisation 26.

C'est une des raisons, avec le fait que la stérilité de la paroi externe n'est généralement pas recherchée, pour lesquelles l'unité de stérilisation 26 vise essentiellement à obtenir la stérilisation du col 16 et de la paroi 15 interne.

Ainsi qu'on l'aura compris, on obtient donc, à la sortie du four 42, des préformes 12 qui sont principalement stériles à l'intérieur et qui doivent avantageusement rester stériles jusqu'à l'opération finale de bouchage.

Selon une variante de réalisation (non représentée) de l'installation 10, une enceinte de confinement stérile peut être prévue pour permettre de contrôler et préserver la stérilité des préformes 12 stérilisées et des bouteilles 14 entre les différentes unités ou postes de l'installation.

Grâce à une installation 10 selon la figure 1, on obtient une réduction logarithmique du nombre de germes de l'ordre de 3D, ou encore 3 Log équivalent à 1000 unités (10³).

De manière connue, la quantité de germes est par exemple dénombrée par comptage après des opérations de lavage, de filtration et de mise en culture.

Selon le mode de réalisation représenté ici, notamment sur la figure 3, les préformes 12 défilent dans le poste de projection 28 de l'unité stérilisation 26 en étant alignées, en position verticale, suivant une direction horizontale longitudinale, dite direction de défilement X1, le col 16 vers le haut.

La direction de défilement X1 passe par les axes A1 des préformes 12 en cours de traitement.

L'axe moyen de projection A2 de la buse 40 est globalement parallèle à l'axe A1 de chaque préforme 12 en cours de traitement et cet axe A2 est excentré radialement, par rapport à l'axe A1 de la préforme 12, d'une valeur de décalage E déterminée.

De préférence, l'axe moyen de projection A2, qui est ici vertical, est excentré suivant un rayon intérieur R1 du col 16 qui est orthogonal à la direction de défilement X1.

Ainsi, la forme de la buse 40 permet de projeter, vers le bas, un flux F de produit stérilisant globalement sous forme de flux laminaire, c'est-à-dire sous la forme d'un rideau vertical longitudinal, à cet effet la buse 40 comporte par exemple une fente longitudinale ou un trou globalement circulaire de projection du flux F.

Le flux F laminaire s'étend ici globalement suivant un plan vertical longitudinal, dit plan de projection X2, qui est décalé radialement, par rapport à la direction de défilement X1, d'une distance égale au décalage E.

Le flux F de produit stérilisant admet ici une infinité d'axes moyens de projection A2 qui s'étendent verticalement dans le plan de projection X2.

De préférence, la valeur de décalage E est comprise entre une valeur minimale Emin sensiblement égale à dix-neuf pourcents du diamètre intérieur D1 du col 16 de chaque préforme 12 et une valeur maximale Emax sensiblement égale à trente-deux pourcents du diamètre intérieur D1 du col 16.

Selon un mode de réalisation avantageux, la valeur de décalage E est choisie fixe et sensiblement égale à huit millimètres, de sorte qu'elle convient à des modèles de préformes 12 possédant des diamètres intérieurs D1 compris environ entre vingt-cinq et quarante deux millimètres.

Grâce à un tel agencement de la buse 40, le flux F de produit stérilisant est sensiblement affleurant avec un premier secteur 60 de la paroi 15 interne de chaque préforme 12, de sorte que le flux F de produit stérilisant lèche ledit secteur 60 de la paroi 15 interne.

En arrivant à l'extrémité inférieure 20 de la préforme 12, le flux F de produit stérilisant glisse sur le fond de la préforme 12 et remonte le long d'un second secteur 62 de paroi 15 interne, diamétralement opposé au premier 60.

Ainsi, le flux F de produit stérilisant balaye globalement l'ensemble de la paroi 15 interne de chaque préforme 12, par écoulement de type laminaire.

Un tel agencement permet notamment d'empêcher la création d'un bouchon de surpression, dans le fond des préformes 12, qui empêcherait le produit stérilisant d'atteindre le fond.

En particulier, la vitesse de propulsion du produit stérilisant, à la sortie de la buse 40, est suffisamment faible pour permettre l'écoulement de type laminaire.

On note que le flux F de produit stérilisant crée un brouillard de produit stérilisant vaporisé qui se diffuse tout autour du flux F, ce qui permet au produit stérilisant de se déposer sur l'ensemble de chaque préforme 12 et ainsi de stériliser ou d'aseptiser simultanément le col 16, la paroi 15 interne et la paroi externe de chaque préforme 12.

On décrira ci-après par comparaison avec l'installation 10 de la figure 1, un deuxième exemple de réalisation d'une installation 10' mettant en oeuvre le procédé de stérilisation de préformes selon l'invention.

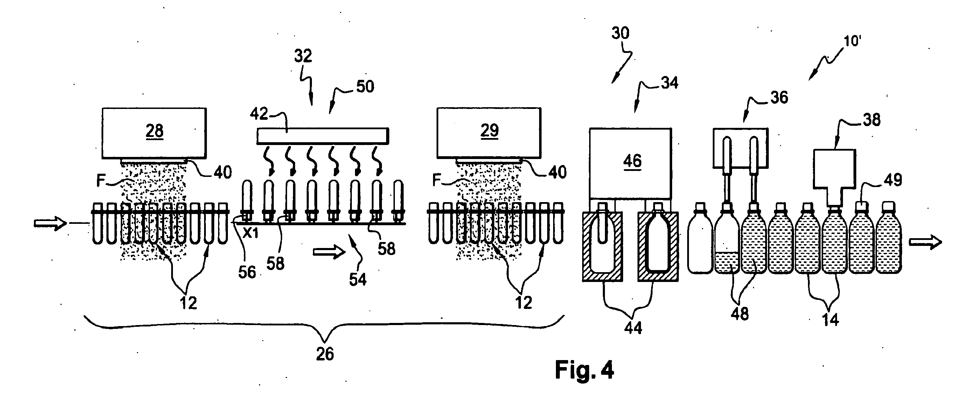

On a représenté à la figure 4, une installation 10' qui est apte à produire des bouteilles stériles par soufflage à partir de préformes stérilisées conformément à l'invention.

Les préformes 12 sont convoyées à l'intérieur de l'installation 10' selon un flot continu qui circule de l'amont vers l'aval, c'est-à-dire de la gauche vers la droite en considérant la figure 4.

L'installation 10' selon la figure 4 diffère principalement de l'installation 10 selon la figure 1 en ce que l'unité de stérilisation 26 comporte un poste de projection 29 supplémentaire, dit deuxième poste.

Le deuxième poste de projection 29 est agencé en aval du poste de conditionnement thermique 32 de l'unité de mise en forme 30 constitué par le four 42 qui assure conjointement la fonction de poste d'activation 50 de l'unité de stérilisation 26.

L'installation 10' comporte, de l'amont vers l'aval, une première unité de stérilisation 26 des préformes 12 comportant un poste de projection 28, dit premier poste ; un poste d'activation 50 formé par le four 42 ; un second poste de projection 29 puis une unité de mise en forme 30 des préformes 12 comportant un poste de conditionnement thermique 32 et un poste de moulage 34.

Avantageusement, l'installation 10' comporte en aval du poste de moulage 34 de l'unité de mise en forme 30, une unité de remplissage 36 et unité de bouchage 38.

Les postes 28, 32, 50 et 34 et les unités 36, 38 sont analogues à celles de la figure 1 décrite précédemment.

De préférence, l'unité de stérilisation 26 comporte des moyens (non représentés) de préparation du produit stérilisant vaporisé pour alimenter le premier poste de projection 28 et/ou le deuxième poste de projection 29.

Le deuxième poste de projection 29 de l'unité de stérilisation 26 est, comme le premier poste 28, pourvu d'au moins une buse 40 qui projette, en cours de traitement, un flux F de produit stérilisant sous la forme d'un jet de vapeur, ici vers le col 16 de chaque préforme 12 à stériliser.

Ainsi, les moyens de préparation du produit stérilisant comportent par exemple des moyens de chauffage (non représentés) du produit stérilisant et une source d'air (non représentée), avantageusement comprimé et/ou stérilisé par tout moyen approprié, qui est prévue pour propulser le produit stérilisant vaporisé à travers la buse 40.

Avantageusement, l'air comprimé est déshydraté et circule à faible vitesse selon un écoulement directif de manière à constituer un vecteur pour la vapeur de produit stérilisant.

De préférence, le produit stérilisant est constitué d'un composé contenant du peroxyde d'hydrogène ou de peroxyde d'hydrogène vaporisé (H₂O₂) qui, au deuxième poste de projection 29, est projeté vers les préformes 12 sous la forme d'un jet de gaz comportant du produit stérilisant à l'état de vapeur, avantageusement un jet de vapeur sèche.

Conformément au procédé de stérilisation selon l'invention, on procède successivement aux étapes (a), (b) et (c) de manière à stériliser au moins la paroi interne 15 et le col 16 de la préforme 12 avec une réduction logarithmique du nombre de germes de l'ordre de 3D, ou encore 3 Log.

Ainsi, on réalise comme précédemment l'étape (b) de dépôt par condensation d'un film uniforme de buée de produit stérilisant au premier poste de projection 28, puis l'étape (c) de chauffage de la préforme 12 au poste d'activation 50 du produit stérilisant.

Au premier poste de projection 28, le flux (F) de produit stérilisant est donc vaporisé sur chaque préforme 12 qui se trouve, conformément à l'étape (a), à une température T1 qui est inférieure à la température de condensation Tc du produit stérilisant.

Au poste d'activation 50 du produit stérilisant, les préformes 12 sont alors au moins chauffées jusqu'à une température T2, de préférence supérieure ou égale à la température de moulage Tm, et à tout le moins supérieure ou égale à la température d'activation Ta.

Avantageusement, le four 42 du poste conditionnement thermique 32 constituant les moyens de chauffage par rayonnement du poste d'activation 50 porte directement chaque préforme 12 à la température de moulage Tm qui est supérieure à la température d'activation Ta du produit stérilisant et ici à la température d'évaporation Te afin de l'éliminer par évaporation.

Avantageusement, le four 42 de chauffage exerce simultanément dans l'installation 10' les fonctions de poste de conditionnement thermique 32 et d'activation 50 tant pour le premier poste de projection 28 que pour le second poste de projection 29.

On décrira ci-après de manière plus détaillée, le deuxième poste de projection 29 de l'unité de stérilisation 26 qui est destiné à traiter les préformes 12 suivant une étape supplémentaire de stérilisation afin d'atteindre, tant sur la paroi interne 15 que sur le col 16, une réduction logarithmique du nombre de germes qui soit supérieure à 3D ou 3 Log.

Après avoir été chauffées par le four 42 des postes 32, 50 à la température T2 déterminée, les préformes 12 passent ensuite directement dans le deuxième poste de projection 29 situé en aval pour y subir l'étape supplémentaire de stérilisation (d).

Avantageusement, la température de chauffage T2 est supérieure ou égale à la température de moulage Tm, par exemple comprise entre quatre vingt quinze degrés Celsius (95°C) et cent trente cinq degrés Celsius (135°C).

Or, comme les préformes 12 sont à une température supérieure à la température d'activation (Ta), par exemple de l'ordre d'environ soixante dix degrés Celsius (70°C) pour le peroxyde d'hydrogène (H₂O₂), il ne se produit pas dans le second poste de projection 29 de dépôt par condensation d'un film uniforme de buée de produit stérilisant comme précédemment dans le premier poste de projection 28.

En effet, le produit stérilisant est instantanément activé et il s'évapore au contact de la préforme chauffée 12 en produisant dès lors un effet bactéricide analogue sur l'ensemble de la préforme 12, c'est-à-dire tant sur la paroi interne 15 et le col 16 que sur les parties externes de la préforme 12.

Grâce à l'étape supplémentaire de stérilisation réalisée au deuxième poste de projection 29, on atteint avantageusement une réduction logarithmique du nombre de germes qui est au moins de l'ordre de 5D ou 5 Log.

Les préformes 12 parcourant l'installation 10, 10' notamment l'unité de stérilisation 26, sont ici orientées verticalement avec le col 16 vers le haut, c'est-à-dire en position dite "col en haut".

En variante, les préformes 12 sont orientées verticalement avec le col 16 vers le bas, soit "col en bas", les préformes 12 pouvant changer d'orientation verticale au sein de l'installation 10, 10', en particulier d'une unité ou d'un poste à l'autre.

L'installation 10, 10' comporte donc ici des moyens de transfert (non représentés) susceptible de procéder au retournement des préformes 12 traitées au poste de projection 28 de l'unité de stérilisation 26 en position "col en haut" à une position "col en bas" pour leur prise en charge par le dispositif de transport 54 associé au four 42.

On note que l'installation 10, 10' a été représentée avec des unités de traitement telles que l'unité de stérilisation 26, l'unité de mise en forme 30, l'unité de remplissage 36, l'unité de bouchage 38.

Ces unités sont alignées, à titre d'illustration, mais ces unités peuvent être agencées selon une configuration différente, notamment avec des dispositifs tournant tels que des carrousels (non représentés).

## Revendications

1. Procédé de stérilisation d'une préforme (12) en matière plastique destinée à être moulée, notamment par soufflage, dans une installation (10, 10') produisant des bouteilles (14) stériles, **caractérisé en ce qu'**il comporte au moins les étapes consistant successivement :
(a) à vérifier que la préforme (12) est à une température (T1) inférieure à la température de condensation (Tc) du produit stérilisant ;
(b) à projeter un flux (F) de produit stérilisant sous forme d'un jet de vapeur vers la préforme (12) de manière à provoquer le dépôt par condensation d'un film de buée (52) de produit stérilisant sensiblement uniforme sur au moins une paroi (15) interne de la préforme à stériliser ; et
(c) à chauffer par rayonnement la préforme (12) ainsi traitée pour la porter à une température (T2) supérieure ou égale à la température d'activation (Ta) du produit stérilisant de manière à stériliser au moins la paroi (15) interne de la préforme.

2. Procédé de stérilisation selon la revendication 1, **caractérisé en ce qu'**il comporte une étape supplémentaire de stérilisation consistant :
(d) à projeter un flux (F) de produit stérilisant sous forme d'un jet de vapeur vers la préforme (12) préalablement chauffée à la température (T2) déterminée.

3. Procédé de stérilisation selon l'une des revendications 1 ou 2, **caractérisé en ce que** la température (T2) de chauffage de la préforme (12) est sensiblement égale à la température de moulage (Tm) par soufflage de la préforme (12) qui est respectivement supérieure à la température d'activation (Ta) et à la température d'évaporation (Te) pour éliminer le produit stérilisant par évaporation.

4. Installation (10, 10') pour produire des bouteilles (14) stériles à partir de préformes (12) en matière plastique mettant en oeuvre le procédé de stérilisation de préformes (12) selon l'une quelconque des revendications précédentes, du type dans lequel les préformes (12) sont convoyées à l'intérieur de l'installation (10, 10') selon un flot continu qui circule de l'amont vers l'aval, et du type comportant successivement au moins :
- une unité de stérilisation (26) comportant au moins un poste de projection (28), dit premier poste, pourvu d'au moins une buse (40) pour projeter, en cours de traitement, un flux (F) de produit stérilisant sous forme d'un jet de vapeur vers chaque préforme (12) à stériliser ;
- une unité de mise en forme (30) comportant un poste de conditionnement thermique (32) qui comporte un four (42) destiné à porter chaque préforme (12) à une température de moulage (Tm), et un poste de moulage (34) apte à produire une bouteille (14) stérile,
**caractérisée en ce que**, au premier poste de projection (28), le flux (F) de produit stérilisant est vaporisé sur chaque préforme (12) dont la température (T1) est inférieure à la température de condensation (Tc) du produit stérilisant de manière qu'un film de buée (52) de produit stérilisant, sensiblement uniforme, se dépose par condensation sur la paroi (15) interne à stériliser, et **en ce que** l'unité de stérilisation (26) comporte un poste d'activation (50) du produit stérilisant comportant des moyens de chauffage par rayonnement constituant le four (42) pour chauffer chaque préforme (12) ainsi traitée jusqu'à une température (T2) supérieure ou égale à la température d'activation (Ta) de manière à stériliser au moins la paroi (15) interne de la préforme (12)
et **en ce que** l'axe moyen de projection (A2) de la buse (40) de vaporisation du produit stérilisant vers le col (16) est globalement parallèle à l'axe (A1) de la préforme (12) en cours de traitement et excentré radialement, par rapport à l'axe (A1) de la préforme (12), de manière à produire un écoulement de produit stérilisant de type laminaire à l'intérieur de la préforme (12).

5. Installation (10, 10') selon la revendication 4, **caractérisée en ce que** les moyens de chauffage du poste d'activation (50) sont constitués par le four (42) du poste de conditionnement thermique (32) de manière que chaque préforme (12) est chauffée à une température de moulage (Tm) qui est respectivement supérieure à la température d'activation (Ta) du produit stérilisant et à la température d'évaporation (Te) pour éliminer le produit stérilisant par évaporation.

6. Installation (10, 10') selon la revendication 5, **caractérisée en ce que** le poste de moulage (34) de l'unité de mise en forme (30) comporte au moins un moule (44) et au moins un dispositif de soufflage (46) associé qui soumet chaque préforme (12) à une surpression interne de manière que chaque préforme (12) stérilisée prenne la forme de l'empreinte du moule (44) pour produire la bouteille (14) stérile.

7. Installation (10, 10') selon la revendication 4, **caractérisée en ce que**, dans l'unité de stérilisation (26), les préformes (12) sont globalement alignées suivant une direction longitudinale de défilement (X1), et sont disposées en position debout, parallèlement les unes à côté des autres, et **en ce que** l'axe de projection (A2) de la buse (40) est décalé radialement suivant une direction sensiblement orthogonale, par rapport à la direction de défilement (X1).

8. Installation (10, 10') selon l'une quelconque des revendications 4 à 7, **caractérisée en ce que** le produit stérilisant est constitué d'un composé contenant du peroxyde d'hydrogène ou de peroxyde d'hydrogène vaporisé qui est activé en chauffant la préforme (12) au-delà de la température d'activation (Ta).

9. Installation (10, 10') selon la revendication 8, **caractérisée en ce que** le produit stérilisant comportant du peroxyde d'hydrogène est projeté à l'état de vapeur à une température supérieure à cent six degrés Celsius.

10. Installation (10, 10') selon l'une quelconque des revendications 4 à 9, du type comportant au moins un dispositif de transport (54) pourvu de moyens de préhension (56) du col (16) de chaque préforme (12) pour convoyer le flot de préformes (12) dans le four (42), **caractérisée en ce que** le dispositif de transport (54) comporte au moins un moyen (58), tel qu'un noyau interne, qui pénètre axialement à l'intérieur du col (16) de chaque préforme (12) en obstruant tout ou partie de l'ouverture intérieure (22) délimitée par le col (16) de manière à accroître le degré de stérilisation par augmentation de la durée d'exposition de la paroi (15) interne de la préforme (12) avec le produit stérilisant.

11. Installation (10') selon l'une quelconque des revendications 4 à 10, **caractérisée en ce que** l'unité de stérilisation (26) comporte, en aval du four (42), un poste de projection (29), dit deuxième poste, qui est destiné à projeter un second flux (F) de produit stérilisant vers chaque préforme préalablement chauffée à une température (T2) déterminée qui est au moins supérieure à la température d'activation (Ta) et à la température d'évaporation (Te) du produit stérilisant.

## Patentansprüche

1. Verfahren zur Sterilisation einer Vorform (12) aus Kunststoff, die insbesondere durch Blasformen in einer Anlage (10, 10') hergestellt wird, die sterile Flaschen (14) produziert, **dadurch gekennzeichnet, dass** sie mindestens die Schritte aufweist, die nacheinander aus folgendem bestehen:
(a) Überprüfen, dass die Vorform (12) eine Temperatur (T1) aufweist, die niedriger ist als die Kondensationstemperatur (Tc) des Sterilisationsproduktes ;
(b) Aufsprühen eines Stromes (F) des Sterilisationsproduktes in Form eines Dampfstrahls auf die Vorform (12), so dass es durch Kondensation zu einem Niederschlag einer im wesentlichen gleichmäßigen, dünnen Nebelschicht (52) des Sterilisationsproduktes an mindestens einer Innenwand (15) der zu sterilisierenden Vorform kommt ; und
(c) Erwärmen der so behandelten Vorform (12) durch Bestrahlung auf eine Temperatur (T2), die höher als oder gleich der Aktivierungstemperatur (Ta) des Sterilisationsproduktes ist, so dass mindestens eine Innenwand (15) der Vorform (12) sterilisiert wird.

2. Verfahren zur Sterilisation nach Anspruch 1, **dadurch gekennzeichnet, dass** es einen zusätzlichen Schritt der Sterilisation aufweist, der aus folgendem besteht :
(d) Aufsprühen eines Stromes (F) des Sterilisationsproduktes in Form eines Dampfstrahls auf die zuvor auf die festgelegte Temperatur (T2) erwärmte Vorform (12).

3. Verfahren zur Sterilisation nach einem der Ansprüche 1 oder 2, **dadurch gekennzeichnet, dass** die Erwärmungstemperatur (T2) für die Vorform (12) im wesentlichen gleich der Temperatur des Blasformens (Tm) der Vorform (12) ist, welche jeweils höher ist als die Aktivierungstemperatur (Ta) und die Verdampfungstemperatur (Te), um das Sterilisationsprodukt durch Verdampfung zu eliminieren.

4. Anlage (10, 10') zur Herstellung von sterilen Flaschen (14) aus Vorformen (12) aus Kunststoff unter Verwendung des Verfahrens zur Sterilisation von Vorformen (12) nach einem der vorhergehenden Ansprüche, bei dem die Vorformen (12) in einem kontinuierlichen Strom, der von vorne bis hinten zirkuliert, in das Innere der Anlage (10, 10') transportiert werden, und welche mindestens folgendes aufweiset :
- eine Sterilisationseinheit (26) mit mindestens einer sogenannten ersten Sprühstation (28), welche mit mindestens einer Düse (40) versehen ist, um während der Behandlung einen Strom (F) des Sterilisationsproduktes in Form eines Dampfstrahls auf jede zu sterilisierende Vorform (12) aufzusprühen;
- eine Formbildungseinheit (30) mit einer Station der thermischen Konditionierung (32), die einen Ofen (42) besitzt, mit dem jede Vorform (12) auf eine Formungstemperatur (Tm) gebracht wird, und eine Formungsstation (34), mit der eine sterile Flasche (14) hergestellt werden kann,
**dadurch gekennzeichnet, dass** der Strom (F) des Sterilisationsproduktes in der ersten Sprühstation (28) an jeder Vorform (12) verdampft, deren Temperatur (T1) niedriger ist als die Kondensationstemperatur (Tc) des Sterilisationsproduktes, so dass sich eine dünne, im wesentlichen gleichmäßige Nebelschicht (52) aus dem Sterilisationsprodukt durch Kondensation auf der zu sterilisierenden Innenwand (15) niederschlägt, und dass die Sterilisationseinheit (26) eine Aktivierungsstation (50) für das Sterilisationsprodukt aufweist, welche Heizelemente durch Bestrahlung besitzt, die den Ofen (42) darstellen, um jede derart behandelte Vorform (12) bis zu einer Temperatur (T2) zu erwärmen, die höher als oder gleich der Aktivierungstemperatur (Ta) ist, so dass mindestens die Innenwand (15) der Vorform (12) sterilisiert wird
und dass die Sprüh-Mittelachse (A2) der Verdampfungsdüse (40) des Sterilisationsproduktes in Richtung Hals (16) während der Behandlung im allgemeinen parallel zu der Achse (A1) der Vorform (12) und radial exzentrisch zur Achse (A1) der Vorform (12) verläuft, so dass es im Innern der Vorform (12) zu einer Laminarströmung des Sterilisationsproduktes kommt..

5. Anlage (10, 10') nach Anspruch 4, **dadurch gekennzeichnet, dass** die Heizelemente der Aktivierungsstation (50) aus dem Ofen (42) der Station der thermischen Konditionierung (32) bestehen, so dass jede Vorform (12) auf eine Formungstemperatur (Tm) erwärmt wird, die jeweils höher ist als die Aktivierungstemperatur (Ta) des Sterilisationsproduktes und die Verdampfungstemperatur (Te), um das Sterilisationsprodukt durch Verdampfen zu eliminieren.

6. Anlage (10, 10') nach Anspruch 5, **dadurch gekennzeichnet, dass** die Formungsstation (34) der Formbildungseinheit (30) mindestens eine Form (44) und mindestens eine zugehörige Blasvorrichtung (46) besitzt, durch die jede Vorform (12) einem internen Überdruck ausgesetzt wird, so dass jede sterilisierte Vorform (12) die Gestalt des Abdrucks der Form (44) zur Herstellung der sterilen Flasche (14) annimmt.

7. Anlage (10, 10') nach Anspruch 4, **dadurch gekennzeichnet, dass** die Vorformen (12) in der Sterilisationseinheit (26) im allgemeinen entlang einer Längsdurchlaufrichtung (X1) ausgerichtet und in einer aufrechten oder stehenden Position parallel nebeneinander angeordnet sind, und dass die Sprühachse (A2) der Düse (40) entlang einer im wesentlichen orthogonalen Richtung gegenüber der Durchlaufrichtung (X1) radial verschoben ist.

8. Anlage (10, 10') nach einem der Ansprüche 4 bis 7,
**dadurch gekennzeichnet, dass** das Sterilisationsprodukt aus einer Verbindung besteht, die Wasserstoffperoxid oder verdampftes Wasserstoffperoxid enthält, das durch Erwärmen der Vorform (12) über die Aktivierungstemperatur (Ta) hinaus aktiviert wird.

9. Anlage (10, 10') nach Anspruch 8, **dadurch gekennzeichnet, dass** das Sterilisationsprodukt, das Wasserstoffperoxid enthält, im Dampfzustand bei einer Temperatur von über einhundertsechs Grad Celsius aufgesprüht wird.

10. Anlage (10, 10') nach einem der Ansprüche 4 bis 9 mit mindestens einer Transportvorrichtung (54), die mit Greifelementen (56) am Hals (16) jeder Vorform (12) versehen ist, um die Menge an Vorformen (12) in den Ofen (42) zu transportieren, **dadurch gekennzeichnet, dass** die Transportvorrichtung (54) mindestens ein Element (58), wie einen internen Kern, besitzt, der axial in das Innere des Halses (16) jeder Vorform (12) eindringt, wobei er die gesamte oder einen Teil der inneren Öffnung (22), die von dem Hals begrenzt wird, blockiert, so dass sich der Grad der Sterilisation dadurch erhöht, dass die Innenwand (15) der Vorform (12) dem Sterilisationsprodukt länger ausgesetzt ist

11. Anlage (10, 10') nach einem der Ansprüche 4 bis 10,
**dadurch gekennzeichnet, dass** die Sterilisationseinheit (26) hinter dem Ofen (42) eine sogenannte zweite Sprühstation (29) besitzt, mit der ein zweiter Strom (F) des Sterilisationsproduktes auf jede Vorform aufgesprüht wird, welche zuvor auf eine festgelegte Temperatur (T2) erwärmt wurde, die mindestens höher als die Aktivierungstemperatur (Ta) und die Verdampfungstemperatur (Te) des Sterilisationsproduktes ist.

## Claims

1. A sterilization method, for sterilizing a plastic preform intended to be molded, especially by blow molding, in an installation (10, 10') producing sterile bottles (14), which method is **characterized in that** it comprises at least the steps consisting, in succession:
(a) in checking that the preform (12) is at a temperature (T1) below the temperature (Tc) of condensation of the sterilizing agent;
(b) in spraying a flow (F) of sterilizing agent in the form of a vapor jet onto the preform (12) so as to cause a substantially uniform film of a mist (52) of the sterilizing agent to be deposited by condensing on at least one internal wall (15) of the preform to be sterilized; and
(c) in radiatively heating the preform (12) thus treated in order to bring it to a temperature (T2) equal to or above the temperature (Ta) for activating the sterilizing agent so as to sterilize at least the internal wall (15) of the preform.

2. The sterilization method as claimed in claim 1, **characterized in that** it includes an additional sterilization step consisting:
(d) in spraying a flow (F) of sterilizing agent in the form of a jet of vapor onto the preform (12) preheated to the specified temperature (T2).

3. The sterilization method as claimed in claim 1 or 2, **characterized in that** the temperature (T2) to which the preform (12) is heated is substantially equal to the temperature (Tm) at which the preform (12) is blow-molded, which temperature is respectively above the activation temperature (Ta) and above the evaporation temperature (Te) in order to eliminate the sterilizing agent by evaporation.

4. An installation (10, 10') for producing sterile bottles (14) from plastic preforms (12) by implementing the method of sterilizing the preforms (12) as claimed in any one of the preceding claims, of the type in which the preforms (12) are conveyed inside the installation (10, 10') as a continuous flow, which runs from the upstream end toward the downstream end, and of the type comprising, in succession, at least:
- a sterilization unit (26) having at least one spray station (28), called the first station, provided with at least one nozzle (40) for spraying, during the treatment, a flow (F) of sterilizing agent in the form of a vapor jet onto each preform (12) to be sterilized; and
- a forming unit (30) comprising a thermal conditioning station (32), which includes an oven (42) intended to bring each preform (12) to a molding temperature (Tm), and a molding station (34) capable of producing a sterile bottle (14),
**characterized in that**, at the first, spray station (28), the flow (F) of sterilizing agent is vaporized on each preform (12), the temperature (T1) of which is below the condensation temperature (Tc) of the sterilizing agent so that a substantially uniform film of a mist (52) of the sterilizing agent is deposited by condensing on the internal wall (15) to be sterilized, and **in that** the sterilization unit (26) includes an activation station (50) for activating the sterilizing agent, comprising radiative heating means constituting the oven (42) for heating each preform (12) thus treated to a temperature (T2) equal to or above the activation temperature (Ta) so as to sterilize at least the internal wall (15) of the preform (12)
and **in that** the mean spray axis (A2) along which the nozzle (40) for vaporizing the sterilizing agent sprays the latter toward the neck (16) is generally parallel to the axis (A1) of the preform (12) during treatment and radially offset relative to the axis (A1) of the preform (12) so as to produce a laminar flow of sterilizing agent inside the preform (12).

5. The installation (10, 10') as claimed in claim 4, **characterized in that** the heating means of the activation station (50) consist of the oven (42) of the thermal conditioning station (32) so that each preform (12) is heated to a molding temperature (Tm) which is respectively above the activation temperature (Ta) of the sterilizing agent and above the evaporation temperature (Te) in order to eliminate the sterilizing agent by evaporation.

6. The installation (10, 10') as claimed in claim 5, **characterized in that** the molding station (34) of the forming unit (30) includes at least one mold (44) and at least one associated blowing device (46) which subjects each preform (12) to an internal overpressure so that each sterilized preform (12) takes the form of the impression of the mold (44) in order to produce the sterile bottle (14).

7. The installation (10, 10') as claimed in claim 4, **characterized in that**, in the sterilization unit (26), the preforms (12) are generally aligned along a longitudinal run direction (X1) and are placed in the upright position, so as to be mutually parallel side by side, and **in that** the spray axis (A2) of the nozzle (40) is radially offset along a direction approximately orthogonal to the run direction (X1).

8. The installation (10, 10') as claimed in any one of claims 4 to 7, **characterized in that** the sterilizing agent consists of a compound containing hydrogen peroxide or vaporized hydrogen peroxide, which is activated by heating the preform (12) above the activation temperature (Ta).

9. The installation (10, 10') as claimed in claim 8, **characterized in that** the sterilizing agent comprising hydrogen peroxide is sprayed in the vapor state at a temperature above one hundred and six degrees Celsius.

10. The installation (10, 10') as claimed in any one of claims 4 to 9, of the type comprising at least one transport device (54) provided with means (56) for gripping the neck (16) of each preform (12) in order to convey the flow of preforms (12) through the oven (42), **characterized in that** the transport device (54) comprises at least one means (58), such as an internal core, which penetrates axially into the neck (16) of each preform (12), partly or completely obstructing the inner opening (22) bounded by the neck (16) so as to increase the degree of sterilization by increasing the time during which the internal wall (15) of the preform (12) is exposed to the sterilizing agent.

11. The installation (10') as claimed in any one of claims 4 to 10, **characterized in that** the sterilization unit (26) includes, downstream of the oven (42), a spray station (29), called the second station, which is intended to spray a second flow (F) of sterilizing agent onto each preform preheated to a defined temperature (T2) which is at least above the activation temperature (Ta) and above the evaporation temperature (Te) of the sterilizing agent.
